# EUROPÄISCHE PATENTSCHRIFT

(11) **EP 3 723 716 B1**
(45) Veröffentlichungstag und Bekanntmachung des Hinweises auf die Patenterteilung: **13.05.2026**
(21) Anmeldenummer: 18806998.3
(22) Anmeldetag: 12.11.2018
(51) Int. Cl.: A61K 8/42, A61K 8/44, A61K 8/46, A61Q 5/02, A61K 8/73, A45D 34/00

(54) **KIT ZUM ERZEUGEN EINES KÖRPERBEHANDLUNGSMITTELS**
KIT FOR PREPARING A BODY TREATMENT PRODUCT
KIT POUR PRÉPARER UN PRODUIT COSMÉTIQUE POUR LE CORPS

(30) Priorität: 14.12.2017 DE 102017222793
(43) Veröffentlichungstag der Anmeldung: 21.10.2020
(73) Patentinhaber: Henkel AG & Co. KGaA, 40589 Düsseldorf (DE)
(72) Erfinder: SCHROEDER, Thomas, 22395 Hamburg (DE); SCHEELE, Soeren, 25421 Pinneberg (DE); METTE, Manuela, 23923 Kleinfeld (DE)
(86) Internationale Anmeldenummer: PCT/EP2018/080869
(87) Internationale Veröffentlichungsnummer: WO 2019/115103

(56) Entgegenhaltungen:
- EP-A1- 0 468 703
- EP-A1- 1 516 613
- EP-A1- 2 932 959
- US-A- 5 643 341
- US-A- 5 929 019
- US-A1- 2002 194 021
- US-A1- 2005 048 021

## Beschreibung

Die Erfindung betrifft ein Kit zum Erzeugen eines Körperbehandlungsmittels, insbesondere eines Haarbehandlungsmittels, und eine Mischvorrichtung zum Erzeugen einer Flüssigkeitsmischung, umfassend ein solches Kit, wie in den Ansprüchen definiert.

In vielen Bereichen des täglichen Lebens gibt es einen anhaltenden Trend hin zu personalisierten Programmen, die auf individuelle Voraussetzungen und Bedürfnisse eines Kunden oder Benutzers eingehen. Dies ist beispielsweise im Bereich der Ernährung oder des Gesundheitswesens der Fall und auch im Bereich der personalisierten Kosmetik. Dies ermöglicht dem Verbraucher oder dem Benutzer von kosmetischen Behandlungsmitteln, individuelle Behandlungsanweisungen und kosmetische Behandlungsmittel zu erhalten, die speziell auf die individuellen Bedürfnisse, beispielsweise seiner Haare oder seiner Haut, zugeschnitten sind und folglich einen besonders hohen Grad an Effektivität ermöglichen.

Ein Friseur kann mit Hilfe seiner Erfahrung und/oder mit geeigneten Analysegeräten den aktuellen Haarzustand (geschädigt, stumpf, brüchig, etc.) eines Kunden bestimmen. Üblicherweise greift er dann bei der Wahl des geeigneten Haarbehandlungsmittels auf im Friseursalon vorhandene Produkte zurück.

Herkömmliche, im Handel erhältliche Haarbehandlungsmittel liegen als begrenzte Produktpalette für einen vorgegebenen Anwendungszweck vor. Beispielsweise stellt jeder Hersteller von Shampoos, Haarkuren oder Haarfarben eine begrenzte Palette an Mitteln bereit. Auch wenn ein Haarzustand eines Nutzers bekannt sein kann, kann es deshalb unmöglich sein, ein optimales Behandlungsergebnis zu erzielen, sondern üblicherweise kann höchstens ein Ergebnis nahe am Wunschergebnis erzielt werden. Siehe z.B. EP 0 468 703 A1 (UNILEVER PLC [GB]; UNILEVER NV [NL]) 29. Januar 1992 (1992-01-29) und US 2002/194021 A1 (MATSUMOTO MITSUO [JP] ET AL) 19. Dezember 2002 (2002-12-19), bzw. EP 1 516 613 A1 (BEIERSDORF AG [DE]) 23. März 2005 (2005-03-23).

Es ist daher wünschenswert, vor Ort, beispielsweise in einem Friseursalon oder an einem Verkaufsort, anhand des ermittelten Haarzustands ein individuell auf den Haarzustand abgestimmtes kosmetisches Haarbehandlungsprodukt für den Nutzer herzustellen.

Ein manuelles Anmischen von unterschiedlichen Haarbehandlungsmitteln, beispielsweise Komponenten für ein Shampoo, eine Spülung, eine Haarkur oder ein Haarfärbemittel, ist jedoch für den Friseur oder den Benutzer selbst eine Herausforderung. Ein optimales Mischergebnis kann nur erreicht werden, wenn die Einzelkomponenten genau in ihren vordefinierten Verhältnissen gemischt werden. Bei einem manuellen Mischen mehrerer Komponenten aus Flaschen kann beispielsweise ein Anteil einer Komponente übermäßig in der Flasche verbleiben, so dass der Mischung ein Anteil der einen Komponente fehlen kann.

Mit Hilfe einer Mischvorrichtung, umfassend eine Mehrzahl von Vorratsbehältern mit dickflüssigen Flüssigkeiten, die Inhaltsstoffe von Körperbehandlungsmitteln enthalten, einer Mehrzahl von Pumpen zum Befördern der dickflüssigen Flüssigkeiten und einem statischen Mischer, ist es möglich, ein gewünschtes Körperbehandlungsmittel exakt und einfach durch Mischen von mindestens zwei Flüssigkeiten herzustellen.

An die Flüssigkeiten in den Vorratsbehältern werden zahlreiche Anforderungen gestellt: so müssen diese gut miteinander mischbar, gut und exakt pumpbar und lagerstabil sein. Außerdem sollten sie hohe Konzentrationen an Wirk- und/oder Hilfsstoffen, wie beispielsweise Öle, Parfüms, enthalten können, so dass auch kleine Menge des gewünschten Haarbehandlungsmittels, beispielsweise für eine einmalige Anwendung, hergestellt werden können.

Es besteht folglich eine Nachfrage an einem Kit, umfassend eine Mehrzahl von Flüssigkeiten, die Inhaltsstoffe von Körperbehandlungsmitteln enthalten, welches bei Einsatz in einer Mischvorrichtung ermöglicht, ein individuelles Körperbehandlungsmittel, insbesondere ein Haarbehandlungsmittel, für einen Nutzer standardisiert und objektiv herzustellen.

Diese Aufgabe wird gelöst, durch ein Kit, umfassend mindestens vier strukturviskose Basisflüssigkeiten zur Herstellung eines Körperbehandlungsmittels, wobei jede Basisflüssigkeit mindestens 10 Gew.-% einer Tensidmischung bestehend aus anionischen Tensiden, amphoteren Tensiden und nichtionischen Tensiden enthält.

Es insbesondere bevorzugt, dass jede Basisflüssigkeit mindestens 10 Gew.-% einer Tensidmischung, bestehend aus Alkylethersulfaten, Alkylamidoalkylbetainen und Alkamidmonoethanolaminen, enthält.

Überaschenderweise hat sich gezeigt, dass Basisflüssigkeiten mit dieser Tensidmischung sehr gut miteinander mischbar sind. Außerdem sind verschiedenste Inhaltsstoffe von Körperbehandlungsmittel mit diesen Tensiden kompatibel und können stabil in die Basisflüssigkeiten eingebracht werden.

Bevorzugte Alkylethersulfate sind ausgewählt aus der Gruppe bestehend aus C₄- bis C₂₄-, bevorzugt C₆- bis C₁₈-, besonders bevorzugt C₈- bis C₁₄-Alkylethersulfaten. Die Alkylreste können linear oder verzweigt sein, wobei lineare bevorzugt sind.

Der Zusatz einer Alkyl-oder Alkenylgruppe mit dem Suffix "eth" beschreibt allgemein die Zugabe von einem oder mehreren Ethylenoxid-Einheiten. Die Anzahl der Ethylenoxid-Einheiten wird durch Zusatz einer ganzen Zahl gekennzeichnet.

Besonders bevorzugte Alkylethersulfate besitzen eine C₁₂-Alkyl-Gruppe (Laurylgruppe) und umfassen Ammonium Laureth Sulfate, Ammonium Laureth-5 Sulfate, Ammonium Laureth-7 Sulfate, Ammonium Laureth-9 Sulfate, Ammonium Laureth-12 Sulfate, DEA-Laureth Sulfate, Magnesium Laureth Sulfate, Magnesium Laureth-5 Sulfate, Magnesium Laureth-8 Sulfate, Magnesium Laureth-16 Sulfate, MEA-Laureth Sulfate, MIPA-Laureth Sulfate, Potassium Laureth Sulfate, Sodium Laureth Sulfate, Sodium Laureth-5 Sulfate, Sodium Laureth-7 Sulfate, Sodium Laureth-8 Sulfate, Sodium Laureth-12 Sulfate, Sodium Laureth-40 Sulfate, TEA-Laureth Sulfate und Mischungen daraus. Ein besonders bevorzugtes Alkylethersulfat ist Sodium Laureth Sulfate (Natriumlaurylethersulfat).

Bevorzugte Alkylamidoalkylbetaine umfassen insbesondere C₄- bis C₂₄-, bevorzugt C₆- bis C₁₈-, besonders bevorzugt C₈- bis C₁₄-Alkylamidopropylbetaine. Die Alkylreste können linear oder verzweigt sein, wobei lineare bevorzugt sind. Besonders bevorzugt enthaltene Alkylamidopropylbetaine sind ausgewählt aus der Gruppe bestehend aus Capryl/Capramidopropyl Betaine, Cocamidopropyl Betaine, Coco/Oleamidopropyl Betaine, Isostearamidopropyl Betaine, Lauramidopropyl Betaine, Oleamidopropyl Betaine, Palmitamidopropyl Betaine, Ricinoleamidopropyl Betaine, Stearamidopropyl Betaine, Tallowamidopropyl Betaine, Undecylenamidopropyl Betaine, Sunfloweramidopropyl Betaineund Mischungen daraus, wobei Cocamidopropyl Betaine (= Cocamidopropyl-Betain) bevorzugt ist.

Bevorzugte Alkamidmonoethanolamine sind ausgewählt aus der Gruppe bestehend aus Cocamide Monoethanolamine (= Cocamide MEA), Oliveamide Monoethanolamine, Palm Kernelamide Monoethanolamine, Palmamide Monoethanolamine, Peanutamide Monoethanolamine, Tallowamide Monoethanolamine, wobei Cocamide Monoethanolamine (N-(Hydroxyethyl)-Kokosfettsäureamid) ganz besonders bevorzugt ist.

Es ist bevorzugt, dass die Menge an Alkylethersulfaten 7 bis 15 Gew.-%, die Menge an Alkylamidoalkylbetainen 1,5 bis 4 Gew.-% und die Menge an Alkamidmonoethanolaminen 1 bis 2 Gew.-%, jeweils bezogen auf die Gesamtmenge an Basisflüssigkeit, beträgt.

Es ist außerdem bevorzugt, dass die Tensidmischung Natriumlaurylethersulfat (INCI: Sodium Laureth Sulfate), Cocamidopropyl-Betain (INCI: Cocamidopropyl Betaine) und N-(Hydroxyethyl)-Kokosfettsäureamid (INCI: Cocamide Monoethanolamine) enthält.

Entsprechend ist es insbesondere bevorzugt, dass die Tensidmischung 7 bis 15 Gew.-% Natriumlaurylethersulfat, 1,5 bis 4 Gew.-% Cocamidopropyl-Betain und 1 bis 2 Gew.-% N-(Hydroxyethyl)-Kokosfettsäureamid jeweils bezogen auf die Gesamtmenge an Basisflüssigkeit, enthält.

Basisflüssigkeiten, enthaltend die Dreierkombination an ausgewählten Tensiden, sind strukturviskos und dadurch sehr gut pumpbar. Die Basisflüssigkeiten lassen sich außerdem gut und exakt dosieren. Dies ermöglicht, die Herstellung von individuellen Körperbehandlungsmitteln in handelsüblichen Mengen, welche von wenigen ml für die Einmal-Anwendung bis hin zu mehreren hundert ml, insbesondere 250 ml oder 400 ml, reichen.

In verschiedenen Ausführungsformen enthält mindestens eine Basisflüssigkeit ferner eine Substanz ausgewählt aus Dialkylsulfosuccinaten.

Bevorzugt in der mindestens einen Basisflüssigkeit enthaltene Dialkylsulfosuccinate sind ausgewählt aus der Gruppe bestehend aus Dialkylsulfosuccinaten, bei denen die Alkylreste jeweils 4 bis 24, bevorzugt 6 bis 18, besonders bevorzugt 6 bis 14, Kohlenstoffatome aufweisen. Die Alkylreste können linear oder verzweigt sein, wobei verzweigte bevorzugt sind. Es können unterschiedliche oder identische Alkylreste in einem Molekül Dialkylsulfosuccinat enthalten sein, wobei identische bevorzugt sind.

Als Gegenion zur Sulfonsäuregruppe können bevorzugt Alkalimetallkationen, Erdalkalikationen oder Ammonium-Ionen, insbesondere Natrium eingesetzt werden. Besonders bevorzugt sind Dialkylsulfosuccinate in den Basisflüssigkeiten enthalten. Die bevorzugt enthaltenen Dialkylsulfosuccinate sind ausgewählt aus der Gruppe bestehend aus Diethylhexyl Sodium Sulfosuccinate, Dinonyl Sodium Sulfosuccinate, Diisononyl Sodium Sulfosuccinate, Dioctyl Sodium Sulfosuccinate, Diheptyl Sodium Sulfosuccinate, Dihexyl Sodium Sulfosuccinate, Dineopentyl Sodium Sulfosuccinate, Diisoamyl Sodium Sulfosuccinate, Dipentyl Sodium Sulfosuccinate, Diamyl Sodium Sulfosuccinate, Dibutyl Sodium Sulfosuccinate, Diisobutyl Sodium Sulfosuccinate, Dicapryl Sodium Sulfosuccinate, Didecyl Sodium Sulfosuccinate, Diundecyl Sodium Sulfosuccinate, Dilauryl Sodium Sulfosuccinate, Dicocoyl Sodium,Sulfosuccinate, Ditridecyl Sodium Sulfosuccinate, Dipropylheptyl Sodium Sulfosuccinate, Dicyclohexyl Sodium Sulfosuccinate, Ammonium Diethylhexyl Sulfosuccinate, Ammonium Dinonyl Sulfosuccinate, Ammonium Diisononyl Sulfosuccinate, Ammonium Dioctyl Sodium Sulfosuccinate, Ammonium Diheptyl Sulfosuccinate, Ammonium Dihexyl Sulfosuccinate, Ammonium Dineopentyl Sulfosuccinate, Ammonium Diisoamyl Sulfosuccinate, Ammonium Dipentyl Sulfosuccinate, Ammonium Diamyl Sulfosuccinate, Ammonium Dibutyl Sulfosuccinate, Ammonium Diisobutyl Sulfosuccinate, Ammonium Dicapryl Sulfosuccinate, Ammonium Didecyl Sulfosuccinate, Ammonium Diundecyl Sulfosuccinate, Ammonium Dilauryl Sulfosuccinate, Ammonium Dicocoyl Sulfosuccinate, Ammonium Ditridecyl Sulfosuccinate, Ammonium Dipropylheptyl Sulfosuccinate, Ammonium Dicyclohexyl Sulfosuccinate, Diethylhexyl Potassium Sulfosuccinate, Dinonyl Potassium Sulfosuccinate, Diisononyl Potassium Sulfosuccinate, Dioctyl Potassium Sulfosuccinate, Diheptyl Potassium Sulfosuccinate, Dihexyl Potassium Sulfosuccinate, Dineopentyl Potassium Sulfosuccinate, Diisoamyl Potassium Sulfosuccinate, Dipentyl Potassium Sulfosuccinate, Diamyl Potassium Sulfosuccinate, Dibutyl Potassium Sulfosuccinate, Diisobutyl Potassium Sulfosuccinate, Dicapryl Potassium Sulfosuccinate, Didecyl Potassium Sulfosuccinate, Diundecyl Potassium Sulfosuccinate, Dilauryl Potassium Sulfosuccinate, Dicocoyl Potassium Sulfosuccinate, Ditridecyl Potassium Sulfosuccinate, Dipropylheptyl Potassium Sulfosuccinate, Dicyclohexyl Potassium Sulfosuccinate, wobei Diethylhexyl Sodium Sulfosuccinate ganz besonders bevorzugt ist.

Es ist bevorzugt, dass die mindestens eine Basisflüssigkeit ferner Diethylhexyl Sodium Sulfosuccinate (Natriumdioctylsulfosuccinat) enthält. Dieses ist beispielsweise unter der Bezeichnung Tego^{®} Sulfosuccinate DO 75 von Evonik erhältlich.

Die Menge an Dialkylsulfosuccinat, insbesondere an Diethylhexyl Sodium Sulfosuccinate, beträgt vorzugsweise 2 bis 20 Gew.-%, mehr bevorzugt 7 bis 15 Gew.-%, jeweils bezogen auf die Gesamtmenge an Basisflüssigkeit.

In verschiedenen Ausführungsformen kann mindestens eine Basisflüssigkeit ferner eine Substanz mit der INCI-Bezeichnung "Guar Hydroxypropyltrimonium Chloride" umfassen, welche einen Stickstoffgehalt von 1,9 bis 2,4 % und/oder einen Molmassenmittelwert im Bereich von 15.000 bis 25.000 Dalton aufweist.

Guar Hydroxypropyltrimonium Chloride ist ein quartäres Trimethylhydroxypropyl-Ammoniumsalz auf Basis von Guarkernmehl und wird Körperbehandlungsmitteln, insbesondere Haarbehandlungsmitteln, als Pflegesubstanz zugesetzt. Aufgrund seiner kationischen Ladung zieht Guar Hydroxypropyltrimonium Chloride auf Haar- oder Hautoberflächen auf oder flockt zusammen mit anwesenden anionischen Tensiden auf Haar- oder Hautoberflächen aus. In beiden Fällen führt dies zu Glättung der Oberfläche und erzeugt eine Erhöhung des Glanzes, eine leichtere Kämmbarkeit, einen Antistatik-Effekt und ein verbessertes Hautgefühl.

Herkömmliches Guar Hydroxypropyltrimonium Chloride kann in Tensid-haltige Zusammensetzungen nur geringen Mengen (bis zu 1 Gew.-%) eingearbeitet werden. Bei höheren Mengen weisen die erhaltenen Zusammensetzungen zu hohe Viskositäten auf und sind nicht mehr pumpbar oder anderweitig verarbeitbar.

Überraschenderweise hat sich gezeigt, dass Guar Hydroxypropyltrimonium Chloride, welches einen Stickstoffgehalt von 1,9 bis 2,4 % und/oder einen Molmassenmittelwert im Bereich von 15.000 bis 25.000 Dalton aufweist, in eine Tensid-haltige Basisflüssigkeit auch in Mengen ≥ 1 Gew.-% eingearbeitet werden kann, ohne nachteilige Effekte auf die Viskosität und damit auf die weitere Verarbeitbarkeit von Tensid-haltigen Basisflüssigkeit zu haben. Guar Hydroxypropyltrimonium Chloride mit einem Stickstoffgehalt von 1,9 bis 2,4 % und einem Molmassenmittelwert im Bereich von 15.000 bis 25.000 Dalton ist beispielsweise unter der Bezeichnung Jaguar^{®} Optima von Solvay erhältlich.

Die Menge an Guar Hydroxypropyltrimonium Chloride, welches einen Stickstoffgehalt von 1,9 bis 2,4 % und/oder einen Molmassenmittelwert im Bereich von 15.000 bis 25.000 Dalton aufweist, beträgt vorzugsweise zwischen 0,02 und 2 Gew.-% und mehr bevorzugt zwischen 0,05 und 1,1 Gew.-%, jeweils bezogen auf das Gesamtgewicht der Basisflüssigkeit.

In verschiedenen Ausführungsformen enthält mindestens eine Basisflüssigkeit ferner mindestens 1 Gew.-%, bezogen auf das Gesamtgewicht der Basisflüssigkeit, einer Substanz mit der INCI-Bezeichnung "Guar Hydroxypropyltrimonium Chloride", welche einen Stickstoffgehalt von 1,9 bis 2,4 % und/oder einen Molmassenmittelwert im Bereich von 15.000 bis 25.000 Dalton aufweist.

In verschiedenen Ausführungsformen weisen die Basisflüssigkeiten eine Viskosität von ≥ 5.000 mPas (Brookfield DV 2+, Spindel 5, 20 U/min, 20°C und 60 Sekunden). Es ist bevorzugt, dass die Basisflüssigkeiten jeweils eine Viskosität in einem Bereich von 5.000 bis 20.000 MPas, aufweisen.

Strukturviskose Flüssigkeiten sind nicht-newtonschen Flüssigkeit, dessen Verformungsverhalten nicht mehr mittels des Newton'schen Gesetzes beschrieben werden kann. Die Viskosität nicht-newtonscher Flüssigkeiten ändert sich insbesondere mit der Schergeschwindigkeit und/oder der Belastungsdauer.

In verschiedenen Ausführungsformen weist das Kit mindestens 8 und ganz besonders bevorzugt mindestens 10 Basisflüssigkeiten auf.

Je mehr Basisflüssigkeiten das Kit umfasst, desto größer ist die Varianz bei den damit herstellbaren Körperoberflächenbehandlungsmitteln. Entsprechend können individuelle Körperbehandlungsmittel hergestellt werden, die optimal auf das Nutzerbedürfnis abgestimmt sind.

In verschiedenen Ausführungsformen weisen die Basisflüssigkeiten ferner jeweils mindestens einen Inhaltsstoff ausgewählt aus der Gruppe bestehend aus Ölen, Parfüms, anorganischen Salzen, organischen Säuren, Anti-Schuppenmittel, Pflegestoffen, Konservierungsmitteln und Farbstoffen enthalten.

Es hat sich überraschenderweise gezeigt, dass insbesondere hohe Konzentrationen an Öl, Parfüm und/oder Pflegestoffe stabil in eine Basisflüssigkeit eingebracht werden können, ohne dass nachteilige Effekte, wie beispielsweise eine stark reduzierte Viskosität, Phasentrennung, oder ähnliches, auftreten, die eine weitere Verarbeitung, beispielsweise ein Mischen in einer Mischvorrichtung, verhindern oder stark erschweren.

Geeignete Öle umfassen beispielsweise Ester der linearen oder verzweigten gesättigten oder ungesättigten Fettalkohole mit 2-30 Kohlenstoffatomen mit linearen oder verzweigten gesättigten oder ungesättigten Fettsäuren mit 2-30 Kohlenstoffatomen, die hydroxyliert sein können. Dazu zählen Cetyl-2-ethylhexanoat, 2-Hexyldecylstearat (zum Beispiel Eutanol^{®}G 16 S), 2-Hexyldecyllaurat, Isodecylneopentanoat, Isononylisononanoat, 2-Ethylhexylpalmitat (zum Beispiel Cegesoft^{®} C 24) und 2-Ethylhexylstearat (zum Beispiel Cetiol^{®} 868). Ebenfalls bevorzugt sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und Ethylenglycoldipalmitat.

Weitere bevorzugte Öle sind ausgewählt aus natürlichen und synthetischen Kohlenwasserstoffen, besonders bevorzugt aus Mineralölen, Paraffinölen, C18-C30-Isoparaffinen ausgewählt.

Weitere bevorzugte Öle sind ausgewählt aus Fettalkoholen mit 6-30 Kohlenstoffatomen, die ungesättigt oder verzweigt und gesättigt oder verzweigt und ungesättigt sind.

Weitere bevorzugte Öle sind ausgewählt aus den Triglyceriden (= Dreifachestern des Glycerins) von linearen oder verzweigten, gesättigten oder ungesättigten, gegebenenfalls hydroxylierten C8-30-Fettsäuren. Besonders bevorzugt kann die Verwendung natürlicher Öle, zum Beispiel Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Haselnussöl, Holundersamenöl, Johannesbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl, und die flüssigen Anteile des Kokosöls und dergleichen sein. Bevorzugt sind aber auch synthetische Triglyceridöle.

Weitere bevorzugte Öle sind ausgewählt aus den Dicarbonsäureestern von linearen oder verzweigten C2-C10-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)succinat.

Weitere bevorzugte Öle sind ausgewählt aus den symmetrischen, unsymmetrischen oder cyclischen Estern der Kohlensäure mit C3-22-Alkanolen, C3-22-Alkandiolen oder C3-22-Alkantriolen, zum Beispiel Dicaprylylcarbonat (Cetiol^{®} CC) oder Glycerincarbonat.

Weitere Öle, die geeignet sind, sind ausgewählt aus den Siliconölen, zu denen zum Beispiel Dialkyl-und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Bevorzugte Siliconöle sind ausgewählt aus höhermolekularen linearen Dimethylpolysiloxanen, im Handel erhältlich zum Beispiel unter der Bezeichnung Dow Corning^{®} 190, Dow Corning^{®} 200 Fluid mit kinematischen Viskositäten (25°C) im Bereich von 5-100 cSt, bevorzugt 5-50 cSt oder auch 5-10 cSt, und Dimethylpolysiloxan mit einer kinematischen Viskosität (25°C) von etwa 350 cSt.

Parfüms, die in Körperbehandlungsmitteln, insbesondere Haarbehandlungsmitteln, eingesetzt werden, umfassen zumeist eine komplexe Mischung aus verschiedenen Riechstoffen. Parfüms können beispielsweise Aldehyd-Riechstoffe, Keton-Riechstoffe, Alkohol-Riechstoffe, Amin-Riechstoffe und/oder Ester-Riechstoffe enthalten.

Die Gesamtmenge an Öl und Parfüm in einer Basisflüssigkeit beträgt vorzugsweise zwischen 0,5 und 15 Gew.-% und mehr bevorzugt zwischen 3 und 10 Gew.-%, jeweils bezogen auf das Gesamtgewicht der Basisflüssigkeit.

Pflegestoffe umfassen insbesondere quaternäre Verbindungen wie monomere kationische oder amphotere Ammoniumverbindungen, monomere Amine, Aminoamide, polymere kationische Ammoniumverbindungen sowie polymere amphotere Ammoniumverbindungen

Geeignete Pflegestoffe umfassen auch Proteinhydrolysate, Vitamine, Provitamine und/oder Vitaminvorstufen.

Die Basisflüssigkeiten können insbesondere auch pH-Stellmittel enthalten. Der pH-Wert der Basisflüssigkeiten liegt vorzugsweise im Bereich von 4 bis 6, mehr bevorzugt im Bereich von 4,5 bis 5,5.

Das Kit kann vorteilhaft in einer Mischvorrichtung zum Erzeugen einer Flüssigkeitsmischung eingesetzt werden. Die erzeugte Flüssigkeitsmischung ist vorzugsweise ein Körperbehandlungsmittel, insbesondere ein Haarbehandlungsmittel, ganz bevorzugt ein Shampoo, Conditioner oder eine Haarkur.

Die Mischvorrichtung zum Erzeugen einer Flüssigkeitsmischung weist vorzugsweise eine Mehrzahl von Vorratsbehältern auf, wobei die Vorratsbehälter eingerichtet sind, eine Mehrzahl von Basisflüssigkeiten aufzunehmen. Ferner weist die Mischvorrichtung vorzugsweise mindestens eine Mischeinheit aus, wobei die Mischeinheit eingerichtet ist, mindestens vier strukturviskose Basisflüssigkeiten aus der Mehrzahl von Vorratsbehältern zu mischen, und wobei die Mischeinheit einen statischen Mischer umfasst. Die Mischvorrichtung enthält vorzugsweise eine Mehrzahl von peristaltischen Pumpen, wobei die peristaltischen Pumpen eingerichtet sind, die Basisflüssigkeiten aus den Vorratsbehältern hin zu der Mischeinheit zu fördern.

Es kann weiterhin vorteilhaft sein, dass die Mischvorrichtung mindestens eine Elektronik aufweist, wobei die Elektronik eingerichtet ist, mindestens eine benutzerspezifische Information bezüglich eines Mischverhältnisses von mindestens zwei Basisflüssigkeiten von einer Computer-App auf mindestens einer mobilen Vorrichtung zu empfangen. Die Mischvorrichtung ist ferner vorteilhaft in der Lage, mittels der Mehrzahl von peristaltischen Pumpen und dem statischen Mischer die mindestens zwei Basisflüssigkeiten gemäß dem vordefinierten Mischverhältnis zu mischen.

### Ausführungsbeispiele

**Tabelle 1: Zusammensetzung von sechs erfindungsgemäßen Basisflüssigkeiten E1 bis E6 (in Gew.-%)**

| Inhaltsstoff (INCI) | E1 | E2 | E3 | E4 | E5 | E6 |
|---|---|---|---|---|---|---|
| Sodium Laureth Sulfate | 8,5 | 8,5 | 12,5 | 8,5 | 8,5 | 8,5 |
| Cocamide MEA | 2,5 | 2,5 | 2,5 | 2,5 | 2,5 | 2,5 |
| Cocamidopropyl Betaine | 1,5 | 0,75 | 1 | 1,5 | 1,5 | 1,5 |
| Sodium Benzoate | 0,5 | 0,5 | 0,5 | 0,5 | 0,5 | 0,5 |
| Disodium Cocoamphoacetate | 0,4 | 0,4 | 0,25 | 0,4 | 0,4 | 0,4 |
| Citric Acid | 0,3 | 0,3 | 0,3 | 0,3 | 0,3 | 0,3 |
| Laureth-4 | 0,2 | 0,2 | 0,2 | 0,2 | 0,2 | 0,2 |
| Sodium Chloride | 0,4 | 0,4 | 0,4 | 0,4 | 0,4 | 0,4 |
| Glycol Distearate | 0,3 | 0,3 | 0,3 | 0,3 | 0,3 | 0,3 |
| Piroctone Olamine | -- | -- | 1,6 | -- | -- | -- |
| PEG-120 Cocoglycolglutamate | -- | -- | -- | -- | 1,2 | 1,2 |
| PEG-40 Hydrogenated Castor Oil | -- | -- | -- | -- | 2,3 | 2,5 |
| Guar Hydroxypropyltrimonium Chloride* | -- | 0,4 | -- | -- | -- | -- |
| Lactic Acid | -- | -- | -- | 1,1 | -- | -- |
| Magnesium Chloride | -- | -- | -- | 2 | -- | -- |
| DOW CORNING^{®} CE-8411** | -- | 1,2 | -- | -- | -- | -- |
| Parfüm | -- | -- | | -- | 2,5 | 3 |
| Farbstoff(e) | -- | -- | | -- | 0,0002 | 0,0006 |
| Aqua | auf 100 | auf 100 | auf 100 | auf 100 | auf 100 | auf 100 |
| pH-Wert | 4,9 | 4,8 | 5,1 | 4,7 | 4,9 | 4,9 |
| Viskosität | 17.600 | 12.400 | 10.100 | 9.700 | 5.600 | 5.100 |

| | | | | | | |
|---|---|---|---|---|---|---|
| * Jaguar C-14 (ex Solvay) ** INCI Name: Bis-Diisopropanolamino-PG-propyl Dimethicone/Bis-Isobutyl PEG-14 Copolymer (and) Polysorbate 20 (and) Butyloctanol | | | | | | |

Zur Herstellung eines Shampoos für Haar, welches nur geringe Haarschädigung aufwies, wurden 60 Teile der Basisflüssigkeit E1, 25 Teile der Basisflüssigkeit E2, 5 Teile der Basisflüssigkeit E4 und 10 Teile der Basisflüssigkeit E5 in einer Mischvorrichtung gemischt. Das erhaltene Shampoo wies eine Viskosität von 8.400 mPas und einen pH-Wert von 4,8 auf.

Zur Herstellung eines Shampoos für Haar, welches einen mittleren Grad an Haarschädigung aufwies, wurden 5 Teile der Basisflüssigkeit E1, 75 Teile der Basisflüssigkeit E2, 10 Teile der Basisflüssigkeit E4 und 10 Teile der Basisflüssigkeit E6 in einer Mischvorrichtung gemischt. Das erhaltene Shampoo wies eine Viskosität von 5.950 mPas und einen pH-Wert von 4,8 auf.

Zur Herstellung eines Shampoos für Haar, welches einen leichten Grad an Haarschädigung und Schuppen aufwies, wurden 40 Teile der Basisflüssigkeit E1, 25 Teile der Basisflüssigkeit E2, 25 Teile der Basisflüssigkeit E3 und 10 Teile der Basisflüssigkeit E5 in einer Mischvorrichtung gemischt. Das erhaltene Shampoo wies eine Viskosität von 7.600 mPas und einen pH-Wert von 5 auf.

**Tabelle 2: Zusammensetzung von sechs erfindungsgemäßen Basisflüssigkeiten E7 bis E12 (in Gew.-%)**

| Inhaltsstoff (INCI) | E7 | E8 | E9 | E10 | E11 | E12 |
|---|---|---|---|---|---|---|
| Sodium Laureth Sulfate | 8,5 | 8,5 | 12,5 | 8,5 | 8,5 | 8,5 |
| Cocamide MEA | 2,5 | 2,5 | 2,5 | 2,5 | 2,5 | 2,5 |
| Cocamidopropyl Betaine | 1,5 | 1,5 | 1 | 1,5 | 1,5 | 1,5 |
| Sodium Benzoate | 0,5 | 0,5 | 0,5 | 0,5 | 0,5 | 0,5 |
| Disodium Cocoamphoacetate | 0,4 | 0,4 | 0,25 | 0,4 | 0,4 | 0,4 |
| Citric Acid | 0,3 | 0,3 | 0,3 | 0,3 | 0,3 | 0,3 |
| Laureth-4 | 0,2 | 0,2 | 0,2 | 0,2 | 0,2 | 0,2 |
| Sodium Chloride | 0,4 | 0,4 | 0,4 | 0,4 | 0,4 | 0,4 |
| Glycol Distearate | 0,3 | 0,3 | 0,3 | 0,3 | 0,3 | 0,3 |
| Tego Sulfosuccinate DO 75*** | -- | 10 | 12 | -- | -- | -- |
| DOW CORNING^{®} CE-8411** | -- | -- | -- | -- | 1,2 | 1,2 |
| Guar Hydroxypropyltrimonium Chloride* | -- | -- | -- | -- | -- | 1 |
| Guar Hydroxypropyltrimonium Chloride**** | -- | -- | -- | -- | 1 | -- |
| PEG-120 Cocoglycolglutamate | 2,0 | -- | -- | 2 | -- | -- |
| PEG-40 Hydrogenated Castor Oil | 2,5 | 2,5 | 2,5 | 2,5 | -- | -- |
| Simmondsia Chinensis (Jojoba) Seed Oil | -- | -- | 1 | 1 | -- | -- |
| Parfüm | 6 | 6 | 6 | 6 | -- | -- |
| Farbstoff(e) | 0,0006 | 0,0006 | 0,0006 | 0,0006 | -- | -- |
| Aqua | auf 100 | auf 100 | auf 100 | auf 100 | auf 100 | auf 100 |
| pH-Wert | 4,9 | 4,8 | 5,1 | 4,7 | 5,0 | 4,9 |
| Viskosität | 3.800 | 7.800 | 6.200 | 1.600 | 11.300 | 29.4000 |

| | | | | | | |
|---|---|---|---|---|---|---|
| * Jaguar C-14 (ex Solvay) ** INCI Name: Bis-Diisopropanolamino-PG-propyl Dimethicone/Bis-Isobutyl PEG-14 Copolymer (and) Polysorbate 20 (and) Butyloctanol *** INCI Name: Diethylhexyl Sodium Sulfosuccinate **** Jaguar Optima (ex Solvay) | | | | | | |

Zur Herstellung eines Shampoos für Haar, welches nur geringe Haarschädigung aufwies, wurden 60 Teile der Basisflüssigkeit E1, 25 Teile der Basisflüssigkeit E2, 5 Teile der Basisflüssigkeit E4 und 10 Teile der Basisflüssigkeit E7 in einer Mischvorrichtung gemischt. Das erhaltene Shampoo wies eine Viskosität von 3.200 mPas auf.

Zur Herstellung eines Shampoos für Haar, welches nur geringe Haarschädigung aufwies, wurden 60 Teile der Basisflüssigkeit E1, 25 Teile der Basisflüssigkeit E2, 5 Teile der Basisflüssigkeit E4 und 10 Teile der Basisflüssigkeit E8 in einer Mischvorrichtung gemischt. Das erhaltene Shampoo wies eine Viskosität von 8.400 mPas auf.

Zur Herstellung eines Shampoos für Haar, welches nur geringe Haarschädigung aufwies, wurden 60 Teile der Basisflüssigkeit E1, 25 Teile der Basisflüssigkeit E2, 5 Teile der Basisflüssigkeit E4 und 10 Teile der Basisflüssigkeit E9 in einer Mischvorrichtung gemischt. Das erhaltene Shampoo wies eine Viskosität von 6.000 mPas auf.

Zur Herstellung eines Shampoos für Haar, welches nur geringe Haarschädigung aufwies, wurden 60 Teile der Basisflüssigkeit E1, 25 Teile der Basisflüssigkeit E2, 5 Teile der Basisflüssigkeit E4 und 10 Teile der Basisflüssigkeit E10 in einer Mischvorrichtung gemischt. Das erhaltene Shampoo wies eine Viskosität von 1.300 mPas auf.

Zur Herstellung eines Shampoos für Haar, welches einen mittleren Grad an Haarschädigung aufwies, wurden 50 Teile der Basisflüssigkeit E1, 10 Teile der Basisflüssigkeit E4, 10 Teile der Basisflüssigkeit E6 und 30 Teile der Basisflüssigkeit E11 in einer Mischvorrichtung gemischt. Das erhaltene Shampoo wies eine Viskosität von 7.900 mPas und einen pH-Wert von 4,8 auf.

Zur Herstellung eines Shampoos für Haar, welches einen mittleren Grad an Haarschädigung aufwies, wurden 5 Teile der Basisflüssigkeit E1, 10 Teile der Basisflüssigkeit E4, 10 Teile der Basisflüssigkeit E6 und 75 Teile der Basisflüssigkeit E12 in einer Mischvorrichtung gemischt. Die Viskosität und der pH-Wert des erhaltenen Shampoos konnten nicht bestimmt werden.

Zur Herstellung eines Shampoos für Haar, welches einen mittleren Grad an Haarschädigung aufwies, wurden 20 Teile der Basisflüssigkeit E1, 10 Teile der Basisflüssigkeit E4, 10 Teile der Basisflüssigkeit E6 und 60 Teile der Basisflüssigkeit E11 in einer Mischvorrichtung gemischt. Das erhaltene Shampoo wies eine Viskosität von 8.200 mPas und einen pH-Wert von 5,0 auf.

Die Viskosität wurde in allen Fällen mit einem Brookfield DV 2+ (Spindel 5, 20 U/min, 20°C und 60 Sekunden) bestimmt.

## Patentansprüche

1. Kit, umfassend mindestens vier strukturviskose Basisflüssigkeiten, strukturviskos wird definiert als nicht-newtonsche Flüssigkeiten, deren Verformungsverhalten nicht mehr mittels des Newton'schen Gesetzes beschrieben werden kann und deren Viskosität sich insbesondere mit der Schergeschwindigkeit und/oder der Belastungsdauer ändert, zur Herstellung eines Körperbehandlungsmittels, wobei jede Basisflüssigkeit mindestens 10 Gew.-% einer Tensidmischung bestehend aus anionischen Tensiden, amphoteren Tensiden und nichtionischen Tensiden enthält.

2. Kit gemäß Anspruch 1, **dadurch gekennzeichnet, dass** jede Basisflüssigkeit mindestens 10 Gew.-% einer Tensidmischung bestehend aus Alkylethersulfaten, Alkylamidoalkylbetainen und Alkamidmonoethanolaminen enthält.

3. Kit gemäß Anspruch 1 oder 2, **dadurch gekennzeichnet, dass** die Menge an Alkylethersulfaten 7 bis 15 Gew.-%, die Menge an Alkylamidoalkylbetainen 1,5 bis 4 Gew.-% und die Menge an Alkamidmonoethanolaminen 1 bis 2 Gew.-%, jeweils bezogen auf die Gesamtmenge an Basisflüssigkeit, beträgt.

4. Kit gemäß einem der vorherigen Ansprüche, **dadurch gekennzeichnet, dass** die Tensidmischung Natriumlaurylethersulfat (INCI: Sodium Laureth Sulfate), Cocamidopropyl-Betain (INCI: Cocamidopropyl Betaine) und N-(Hydroxyethyl)-Kokosfettsäureamid (INCI: Cocamide Monoethanolamine) enthält.

5. Kit gemäß einem der vorherigen Ansprüche, **dadurch gekennzeichnet, dass** mindestens eine Basisflüssigkeit ferner eine Substanz ausgewählt aus Dialkylsulfosuccinaten enthält.

6. Kit gemäß Anspruch 5, **dadurch gekennzeichnet, dass** die Substanz Natriumdioctylsulfosuccinat (INCI: Diethylhexyl Sodium Sulfosuccinate) ist.

7. Kit gemäß einem der vorherigen Ansprüche, **dadurch gekennzeichnet, dass** mindestens eine Basisflüssigkeit ferner eine Substanz mit der INCI-Bezeichnung "Guar Hydroxypropyltrimonium Chloride", welche einen Stickstoffgehalt von 1,9 bis 2,4 % und/oder einen Molmassenmittelwert im Bereich von 15.000 bis 25.000 Dalton aufweist, umfasst.

8. Kit gemäß einem der vorherigen Ansprüche, **dadurch gekennzeichnet, dass** es mindestens 8 und ganz besonders bevorzugt mindestens 10 Basisflüssigkeiten aufweist.

9. Kit gemäß einem der vorherigen Ansprüche, **dadurch gekennzeichnet, dass** die Basisflüssigkeiten eine Viskosität von ≥ 5.000 mPas (Brookfield DV 2+, Spindel 5, 20 U/min, 20°C und 60 Sekunden) aufweisen.

## Claims

1. A kit comprising at least four structurally viscous base liquids for the preparation of a body treatment agent, wherein each base liquid contains at least 10% by weight of a surfactant mixture consisting of anionic surfactants, amphoteric surfactants, and nonionic surfactants.

2. A kit according to claim 1, **characterized in that** each base liquid contains at least 10% by weight of a surfactant mixture consisting of alkyl ether sulfates, alkylamidoalkyl betaines, and alkamide monoethanolamines.

3. A kit according to claim 1 or 2, **characterized in that** the amount of alkyl ether sulfates is 7 to 15% by weight, the amount of alkylamidoalkyl betaines is 1.5 to 4% by weight, and the amount of alkamide monoethanolamines is 1 to 2% by weight, in each case based on the total amount of base liquid.

4. A kit according to any of the preceding claims, **characterized in that** the surfactant mixture contains sodium lauryl ether sulfate (INCI: Sodium Laureth Sulfate), cocamidopropyl betaine (INCI: Cocamidopropyl Betaine), and N-(hydroxyethyl)cocoyl amide (INCI: Cocamide Monoethanolamine).

5. A kit according to any of the preceding claims, **characterized in that** at least one base liquid further contains a substance selected from dialkyl sulfosuccinates.

6. A kit according to claim 5, **characterized in that** the substance is sodium dioctyl sulfosuccinate (INCI: Diethylhexyl Sodium Sulfosuccinate).

7. A kit according to any of the preceding claims, **characterized in that** at least one base liquid further comprises a substance with the INCI designation "Guar Hydroxypropyltrimonium Chloride," which has a nitrogen content of 1.9 to 2.4% and/or a number-average molecular weight in the range of 15,000 to 25,000 daltons.

8. A kit according to any of the preceding claims, **characterized in that** it comprises at least 8 and, most preferably, at least 10 base liquids.

9. A kit according to any of the preceding claims, **characterized in that** the base liquids have a viscosity of ≥ 5,000 mPas (Brookfield DV 2+, spindle 5, 20 rpm, 20°C, and 60 seconds).

## Revendications

1. Kit comprenant au moins quatre liquides de base à viscosité structurale destinés à la fabrication d'un produit de soin corporel, chaque liquide de base contenant au moins 10 % en poids d'un mélange de tensioactifs composé de tensioactifs anioniques, de tensioactifs amphotères et de tensioactifs non ioniques.

2. Kit selon la revendication 1, **caractérisé en ce que** chaque liquide de base contient au moins 10 % en poids d'un mélange de tensioactifs composé d'alkyléthersulfates, d'alkylamidoalkylbétaïnes et d'alkamidmonoéthanolamines.

3. Kit selon la revendication 1 ou 2, **caractérisé en ce que** la quantité d'alkyléthersulfates est comprise entre 7 et 15 % en poids, la quantité d'alkylamidoalkylbétaïnes est comprise entre 1,5 et 4 % en poids et la quantité d'alkamidmonoéthanolamines est comprise entre 1 et 2 % en poids, dans chaque cas par rapport à la quantité totale de liquide de base.

4. Kit selon l'une des revendications précédentes, **caractérisé en ce que** le mélange tensioactif contient du lauryléthersulfate de sodium (INCI: Sodium Laureth Sulfate), de la cocamidopropylbétaïne (INCI: Cocamidopropyl Betaine) et de l'amide d'acide gras de coco N-(hydroxyéthyl) (INCI: Cocamide Monoethanolamine).

5. Kit selon l'une des revendications précédentes, **caractérisé en ce qu'**au moins un liquide de base contient en outre une substance choisie parmi les sulfosuccinates de dialkyle.

6. Kit selon la revendication 5, **caractérisé en ce que** la substance est le dioctylsulfosuccinate de sodium (INCI: Diethylhexyl Sodium Sulfosuccinate).

7. Kit selon l'une des revendications précédentes, **caractérisé en ce qu'**au moins un liquide de base comprend en outre une substance portant la désignation INCI "Guar Hydroxypropyltrimonium Chloride", qui présente une teneur en azote de 1,9 à 2,4 % et/ou une masse moléculaire moyenne comprise entre 15 000 et 25 000 daltons.

8. Kit selon l'une des revendications précédentes, **caractérisé en ce qu'**il comprend au moins 8 et, de manière particulièrement préférée, au moins 10 liquides de base.

9. Kit selon l'une des revendications précédentes, **caractérisé en ce que** les liquides de base présentent une viscosité d' ≥ 5 000 mPas (Brookfield DV 2+, broche 5, 20 tr/min, 20 °C et 60 secondes).
